Europäisches Patentamt

European Patent Office

Office européen des brevets

(19)

(11) Numéro de publication: **0 185 573 B1**

(12) **FASCICULE DE BREVET EUROPEEN**

(45) Date de publication de fascicule du brevet: **20.05.92**

(51) Int. Cl.5: **C12N 15/36**, C12N 15/86, A61K 39/29, C12N 5/00

(21) Numéro de dépôt: **85402261.3**

(22) Date de dépôt: **20.11.85**

(54) Expression et sécrétion de polypeptides dans des eucaryotes, sous contrôle d'un promoteur d'adénovirus.

(30) Priorité: **20.11.84 FR 8417674**

(43) Date de publication de la demande:
**25.06.86 Bulletin 86/26**

(45) Mention de la délivrance du brevet:
**20.05.92 Bulletin 92/21**

(84) Etats contractants désignés:
**AT BE CH DE FR GB IT LI LU NL SE**

(56) Documents cités:
**EP-A- 0 086 707**
**WO-A-83/02393**

**PROC. NATL. ACAD. SCI, vol. 80, décembre 1983, pages 7586-7590, US; I. KRUCZEK et al.: "Expression of the chloramphenicol acetyl-transferase gene in mammalian cells under the control of adenovirus type 12 promoters: Effect of promoter methylation on gene ex-pression"**

(73) Titulaire: **INSTITUT PASTEUR**
**25-28, rue du Docteur Roux**
**F-75724 Paris Cédex 15(FR)**

Titulaire: **CENTRE NATIONAL DE LA RECHER-CHE SCIENTIFIOUE (CNRS)**
**15, Ouai Anatole France**
**F-75700 Paris Cedex 07(FR)**

Titulaire: **INSTITUT NATIONAL DE LA SANTE ET DE LA RECHERCHE MEDICALE (INSERM)**
**101, rue de Tolbiac**
**F-75654 Paris Cédex 13(FR)**

(72) Inventeur: **Perricaudet, Michel**
**6 rue du Renard**
**F-75004 Paris(FR)**
Inventeur: **Tiollais, Pierre**
**16 rue de la Glacière**
**F-75013 Paris(FR)**
Inventeur: **Levrero, Massimo**
**199 via Alessandria**
**F-00198 Rome(IT)**

EP 0 185 573 B1

MOLECULAR AND CELLULAR BIOLOGY, vol. 3, no. 1, janvier 1983, pages 44-55, American Society for Microbiology; C.W. CROWLEY et al.: "Plasmid-directed synthesis of hepatitis B surface antigen in monkey cells"

PROC. NATL. ACAD. SCI, vol. 81, no. 24, décembre 1984, pages 7708-7712, Washington, US; M.-L. MICHEL et al.: "Synthesis in animal cells of hepatitis B surface antigen particles carrying a receptor for polymerized human serum albumin"

NUCLEIC ACIDS RESEARCH, vol. 11, no. 24, 1983, pages 8735-8745, IRL Press Ltd, Oxford, GB; P. SASSONE-CORSI et al.: "Far upstream sequences are required for efficient transcription from the adenovirus-2 E1A transcription unit"

CHEMICAL ABSTRACTS, vol. 96, 1982, page 123, résumé no. 63555n, Columbus, Ohio, US; A. FIRE et al.: "In vitro transcription of adenovirus"; & J. VIROL. 1981, 40(3), 703-19

CHEMICAL ABSTRACTS, vol. 102, 1985, page 158, résumé no. 18553s, Columbus, Ohio, US; J.R. NEVINS et al.: Cis and trans acting regulation of early adenovirus transcription"; & TRANSFER EXPRESSION EUKARYOTIC GENES [PROC. - SYMP.] 1983 (Pub. 1984), 239-45

CHEMICAL ABSTRACTS, vol. 98, 1983, page 191, résumé no. 28934e, Columbus, Ohio, US; K. ODA et al.: "Expression of adenovirus type 12 E1A gene in monkey cells, using a simian virus 40 vector"; & J. VIROL. 1983, 45(1), 408-19

Nuclear Acids Research vol. 12 n0.4

Cell vol.23 pp 825-36 (1981)

Inventeur: **Ballay, Annick**
**646 rue de Montamets**
**F-78630 Orgeval(FR)**

(74) Mandataire: **Gutmann, Ernest et al**
**S.C. Ernest Gutmann - Yves Plasseraud 67,**
**boulevard Haussmann**
**F-75008 Paris(FR)**

## Description

L'invention concerne un ADN recombinant comportant une séquence nucléotidique codant pour un polypeptide détermine sous le contrôle d'un promoteur d'adénovirus, des vecteurs contenant cet ADN recombinant et des cellules eucaryotes transformées par cet ADN recombinant, les produits d'excrétion de ces cellules transformées et leurs applications, notamment à la constitution de vaccins.

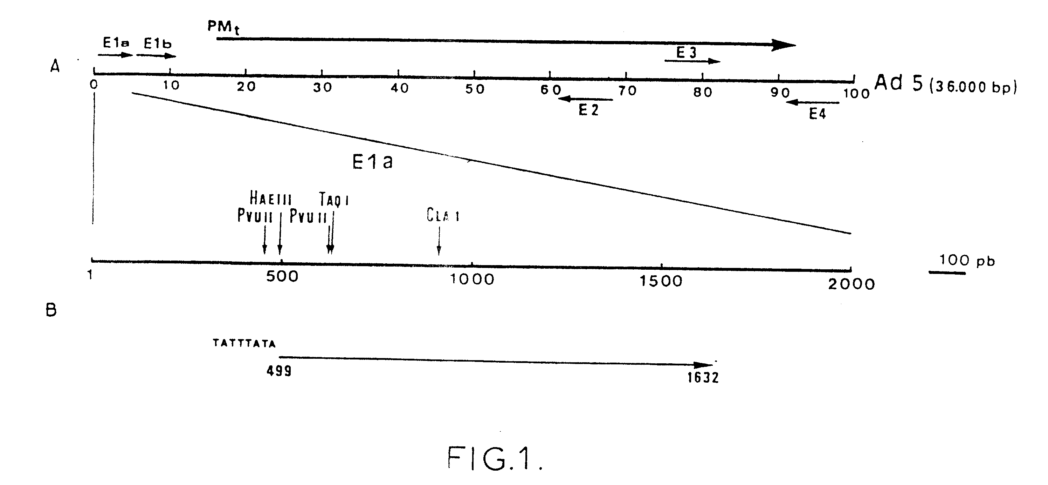

Les adénovirus humains possèdent un long génome (environ 36.000 pb) linéaire et double brin qui code pour au moins 30 protéines. Le cycle viral, au cours de l'infection de cellules permissives, est divisé en deux phases, précoce et tardive. Il est connu que les quatre régions du génome viral exprimées en phase précoce sont appelées régions E1, E2, E3 et E4, dont les positions respectives dans le génome viral entier sont schématiquement représentées dans la fig. 1. La région E1, située à l'extrémite gauche du génome, est elle-même divisée en deux régions E1A et E1B. Le passage de la phase précoce à la phase tardive, marque par la réplication du DNA viral, est caractérisé par un changement brutal du programme génétique du virus. L'expression de certains gènes précoces est réprimée alors que la transcription des gènes tardifs s'effectue principalement à partir d'un seul promoteur, le promoteur majeur tardif (fig. 1). De plus, on observe une forte répression de la synthèse des protéines de la cellule hôte (1).

L'organisation génétique des adénovirus humains de type 2 ou 5 (Ad2, Ad5) est suffisamment connue pour que l'on puisse manipuler leur génome in vitro et son utilisation comme vecteur d'expression d'un gène étranger dans une cellule animale en culture a déjà été envisagée. Il est en effet connu que la région E3, qui représente 6 % du génome, n'est pas essentielle in vitro et peut donc être substituée dans sa totalité (2). La taille du fragment de DNA étranger qu'il est possible d'insérer dans le génome de ces virus est grande. En effet, le virus peut encapsider un génome dont la longueur excède de 5 % celle du génome sauvage.

Le promoteur de la région précoce E1A d'un adénovirus, transporté dans des cellules de souris dans lesquelles la réplication des rétrovirus n'est pas possible, s'est avéré pouvoir y exercer quand même son activité promotrice. Ainsi un gène procaryote codant pour la chloramphénicol-acétyltransférase at'il pu être exprimé dans un tel système cellulaire sous le contrôle dudit promoteur (Kruczek I. et al (1983), Proc. Natl. Acad. Sci. USA).

Différents vecteurs dérivés des adénovirus de type 2 ou 5 ont donc été construits. Dans ces recombinants, le gène étranger était exprimé sous le contrôle du promoteur majeur tardif. Ceci a permis d'obtenir dans certains cas une synthèse de la protéine codée par un gène étranger à un niveau comparable à celui des protéines virales tardives (4, 5, 6, 7), avec des efficacités de traduction in vivo faibles, notamment eu égard aux efficacités de transcription en ARNs également observées (6). En outre l'expression du gène étranger sous le contrôle du promoteur tardif ne peut se manifester que dans la phase tardive du cycle viral.

L'invention découle de la constatation que le promoteur de la région précoce E1A du génome d'un adénovirus (ci-après désigné simplement par "promoteur E1A") pouvait contrôler de façon particulièrement efficace l'expression dans un vecteur viral d'un gène hétérologue (c'est-à-dire étranger vis-à-vis des gènes qui lui sont normalement associés dans l'adénovirus) ou plus généralement d'une séquence nucléotidique hétérologue codant pour une séquence polypeptidique dont l'expression est recherchée. Dans les conditions définies ci-après, le promoteur E1A se comporte comme un promoteur fort, et ce plus particulièrement lorsque l'ensemble promoteur E1A-séquence codante hétérologue est inséré dans ce vecteur viral.

L'invention découle de la constatation que le promoteur de la région précoce E1A du génome d'un adénovirus (ci-après désigné simplement par "promoteur E1A") pouvait contrôler de façon particulièrement efficace l'expression dans un vecteur viral d'un gène hétérologue (c'est-à-dire étranger vis-à-vis des gènes qui lui sont normalement associés dans l'adénovirus) ou plus généralement d'une séquence nucléotidique hétérologue codant pour une séquence polypeptidique dont l'expression est recherchée. Dans les conditions définies ci-après, le promoteur E1A se comporte comme un promoteur fort, et ce plus particulièrement lorsque l'ensemble promoteur E1A-séquence codante hétérologue est inséré dans ce vecteur viral.

L'invention concerne donc de façon générale un vecteur recombinant pour la transformation de lignées cellulaires eucaryotes, notamment humaines ou animales, choisies parmi celles qui sont infectables par des adénovirus ou dont les polymérases endogènes sont susceptibles de reconnaître les promoteurs des adénovirus, ce vecteur étant en outre modifié par un acide nucléique d'insertion contenant une séquence nucléotidique codant pour une séquence polypeptidique dont l'expression dans lesdites lignées cellulaires est recherchée, caractérisé en ce que ladite séquence d'insertion est substituée aux gènes E1A normalement placés sous le contrôle direct dudit promoteur précoce de la région E1A, cette séquence d'insertion étant elle-même placée sous le contrôle direct de ce promoteur et en ce qu'il comporte, en aval de l'acide nucléique d'insertion, l'ensemble des séquences essentielles d'un génome d'adénovirus qui sont nécessai-

3

res à la réplication de l'adénovirus correspondant et qui sont normalement situées en aval des gènes normalement sous le contrôle direct du promoteur précoce E1A dans ledit génome, et en ce que la susdite séquence nucléotidique codant pour la séquence polypeptidique dont l'expression est recherchée est hétérologue vis-à-vis des gènes du susdit adénovirus.

S'agissant d'un vecteur viral, dérivé d'un adénovirus, on peut alors également bénéficier de l'avantage s'attachant à la région E1A des adénovirus, à savoir que son expression est constitutive et permanente tout au long du cycle viral (8, 9).

Une forme particulière préférée de l'ADN recombinant selon l'invention est caractérisée par le fait qu'elle comporte, en aval de l'acide nucléique d'insertion, dans le sens de la transcription, un génome défectif d'adénovirus comprenant néanmoins l'ensemble de celles des séquences essentielles nécessaires à la réplication de l'adénovirus correspondant, qui sont normalement situées en aval des gènes normalement sous le contrôle direct du promoteur précoce E1A dans ledit génome.

Avantageusement, le génome défectif d'adénovirus avec lequel l'ADN recombinant conforme à l'invention est associé, est constitué par un génome complet d'adénovirus, cependant dépourvu de la partie antérieure de la région E1A du génome viral, notamment de son fragment 0-2,6 % (pourcentage exprimé par rapport à la taille totale du génome de l'adénovirus).

Les ADNs recombinants de l'invention, associés avec des éléments de vecteurs tels que ceux qui ont été mentionnés ci-dessus, constituent en fait des vecteurs contenant lesdits ADNs recombinants. Il sera encore en ce qui les concerne fait référence à des "virus recombinants défectifs", lorsque les éléments de vecteurs associés à l'ADN-recombinant selon l'invention seront dérivés d'un génome défectif d'adénovirus. Ces virus recombinants défectifs sont avantageusement utilisés pour la transformation de lignées cellulaires transformables d'eucaryotes supérieurs (notamment d'origine humaine ou animale) comportant elles-mêmes une séquence distincte de nucléotides apte à complémenter la partie du génome de l'adénovirus dont le susdit vecteur est dépourvu, ladite séquence distincte étant de préférence incorporée au génome des cellules de ladite lignée cellulaire.

A titre d'exemple préféré de telles lignées cellulaires, on mentionnera la lignée 293, lignée de rein embryonnaire humain qui contient, intégrés dans son génome, les onze premiers pourcents de l'extrémité gauche du génome d'un Ad5. Ceux-ci permettent de complémenter des virus recombinants defectifs qui portent des délétions de cette région (10).

L'utilisation de ces systèmes vecteur virus défectif recombinant - cellules contenant une séquence capable de complémenter les virus recombinants défectifs, est d'un intérêt tout particulier, lorsque la séquence nucléotidique contenue dans l'acide nucléique d'insertion de l'ADN recombinant code pour une protéine qui, lorsqu'elle est exprimée dans un hôte cellulaire naturel sous le contrôle de son promoteur naturel, est excrétée dans le milieu d'une culture de cet hôte cellulaire naturel.

Le gène S du génome du virus de l'hépatite B constitue à cet égard une séquence nucléotidique d'un intérêt particulier, et ce pour plusieurs raisons. D'une part, le produit d'expression du gène S dans les cellules qui l'expriment, l'HBsAg (11, 12), est secrété dans le surnageant cellulaire sous forme de particules faciles à détecter et à quantifier par dosage radioimmunologique, ce qui permet une évaluation précise de la capacité d'expression du vecteur viral. D'autre part, l'invention fournit un vecteur viral recombinant permettant l'étude de l'expression des gènes de l'HBV tant au niveau de la transcription que de la traduction, ce qui est d'autant plus intéressant qu'il n'existait pas jusqu'à ce jour de système de culture cellulaire capable de propager le virus de l'hépatite B (HBV). Enfin, l'infection cellulaire par le virus recombinant adénovirus-HBV illustre de façon particulièrement avantageuse la base méthodologique d'un procédé de fabrication d'un vaccin contre un agent pathogène déterminé (en l'occurrence le virus de l'hépatite B dans l'exemple considéré).

Une autre séquence nucléotidique du génome du virus de l'hépatite B d'un intérêt particulier est le gène S muni de sa région pré-S2 qui code pour l'antigène HBs et pour un recepteur de la sérumalbumine humaine polymérisée (pHSA)(25),(26).

Il va de soi que l'on peut substituer dans l'ADN recombinant le gène S par toute autre séquence nucléotidique codant pour un antigène protecteur distinct contre un autre agent pathogene déterminé, surtout lorsque cet antigène protecteur distinct est lui-même normalement susceptible d'être secrété par les cellules transformées par l'ADN recombinant. Il va de soi également que l'on peut substituer dans l'ADN recombinant le gène S et la région pré-S2 par toute autre séquence nucléotidique codant pour un antigène protecteur distinct contre un autre agent pathogène déterminé, surtout lorsque cet antigène protecteur distinct est lui-même normalement susceptible d'être sécrété par les cellules transformées par l'ADN recombinant. La séquence nucléotidique codant pour cet antigène protecteur distinct peut d'ailleurs éventuellement être insérée dans l'ADN recombinant en phase avec un autre gène, par exemple l'antigène HBsAg, dès lors que cet autre gène peut être utilisé comme "locomotive" pour promouvoir l'excrétion également de cet antigène distinct, notamment sous forme de protéine hybride. Au titre des antigènes distincts susceptibles d'être ainsi produits (le cas échéant sous forme de protéine hybride), on mentionne par exemple des glycoprotéines de structure du virus d'Epsteim-Barr.

Les premiers nucléotides de la séquence nucléotidique codant pour un polypeptide déterminé (protéine "simple" ou hybride) sont placés, notamment par construction aussi près que possible de ce promoteur, notamment de la "boite TATA" (TATA box), caractéristique du promoteur, étant cependant entendu que la séquence nucléotidique entre le promoteur et l'ATG initiateur de la séquence nucléotidique codant pour ledit polypeptide déterminé devra en général contenir les triplets codant pour l'extrémité 5' non traduite de l'ARN messager correspondant normalement à la séquence codante et contenant les séquences d'appariement aux ribosomes nécessaires à une traduction efficace. Cette extrémité 5' non traduite de l'ARN messager peut d'ailleurs être remplacée par l'extrémité 5' non traduite d'un ARN messager distinct de celui normalement associé a une séquence codante déterminée. Par exemple, on peut, dans le cas du gène S, remplacer l'extrémité 5' non traduite contenant le gène pré-S ou jouxtant celui-ci par l'extrémité 5' non traduite de l'ARN messager de l'antigène T de SV40. Mais on a aussi remarqué que lorsqu'on utilise une séquence d'ADN contenant les régions S et pré-S2 du génome du virus de l'hépatite B sous le contrôle du promoteur fort E1A, il est possible d'obtenir les expressions à la fois de la région pré-S2 et de la région S. Toute autre extrémité 5' non traduite d'ARN messager peut être utilisée, dès lors qu'elle est compatible avec l'autre extrémité similaire choisie.

Il est avantageux que la distance entre la boîte TATA du promoteur et le site d'initiation de l'ARN messager soit d'environ 30 nucléotides.

Le promoteur E1A de l'ADN recombinant selon l'invention et encore plus généralement le vecteur selon l'invention mettant en oeuvre des parties plus importantes du génome d'un adénovirus sont de préférence issus d'un adénovirus appartenant à la catégorie C, telle qu'elle a été définie par TOOZE. Ces adénovirus ont la propriété connue de n'être pas oncogènes. Les sous-types Ad2 ou Ad5 de cette catégorie d'adénovirus se caractérisent par un pouvoir transformant important. L'utilisation de ce dernier type d'ADN recombinant est donc particulièrement recommandée, lorsque le produit d'expression recherché est destiné à la production d'antigènes protecteurs, notamment de principes actifs de vaccins. Cela sera encore d'autant plus vrai dans le cas où des adénovirus entiers, et même infectieux, seront utilisés comme principes actifs de vaccins vivants, notamment dans les conditions qui seront encore explicitées plus loin.

L'invention concerne naturellement également les lignées cellulaires, notamment d'origine humaine ou animale, qui sont transformées par des ADN recombinants tels que définis ci-dessus et qui ont été rendues capables de synthétiser un polypeptide codé par la séquence nucléotidique (ou lesdites séquences nucléotidiques) contenues dans ces ADNs recombinants et placées sous le contrôle direct dudit promoteur.

L'invention concerne plus particulièrement encore les lignées cellulaires transformées avec un vecteur recombinant conforme à l'invention et en outre caractérisées en ce que les cellules de ces lignées cellulaires contiennent elles-mêmes une séquence distincte de nucléotides aptes à complémenter la partie du génome de l'adénovirus dont le susdit vecteur est dépourvu, ladite séquence distincte étant de préférence incorporée au génome des cellules de ladite lignée cellulaire.

A ce titre, la lignée 293 déjà mentionnée plus haut, après avoir été transformée par les vecteurs recombinants, constitue une culture cellulaire préférée selon l'invention. Grâce à la séquence de complémentation que contiennent les cellules de cette lignée, on observe une multiplication virale importante à l'intérieur de ces cellules et, par voie de conséquence, une expression également multipliée de la séquence codante pour le polypeptide prédéterminé. Dans le premier cas où cette séquence codante est le gène S, on obtient une production importante d'antigènes HBsAg excrétés dans le milieu de culture de ces cellules. Dans le second cas où les séquences codantes sont constituées par le gène S et la région pré-S2 du virus de l'hépatite B, l'adénovirus recombinant dirige in vitro la synthèse des particules HBsAg possédant une activité de récepteur pour la pHSA. Injecté à des lapins, ce virus recombinant produit des anticorps anti-HBsAg et anti-pHSA. Ceci montre la possibilité d'utiliser un adénovirus recombinant pour exprimer un gène à la fois in vitro et in vivo. Les mêmes vecteurs peuvent être utilisés pour la transformation de cellules Vero dans des conditions analogues.

Les vecteurs recombinants selon l'invention peuvent également être utilisés pour la transformation de cellules ne possédant pas elle-même la séquence de complémentation dans les conditions qui ont été indiquées ci-dessus. Il pourra alors être nécessaire de procéder à une co-transformation de ces derniers types de cellules, d'une part, avec le vecteur recombinant selon l'invention, d'autre part, avec un adénovirus non défectif ou un ADN recombinant distinct contenant les séquences d'adénovirus dont est dépourvu le vecteur recombinant conforme à l'invention. Il pourra certes être observé dans ce dernier cas une production simultanée d'antigènes HBsAg (lorsque la séquence codante contient le gène S) et de l'adénovirus répliqué et libéré par les cellules ainsi transformées. L'antigène protecteur formé peut

EP 0 185 573 B1

cependant, le cas échéant, être séparé de la suspension virale, par exemple par mise en contact du milieu de culture avec des anticorps anti-adénovirus, de préférence immobilisés sur un support solide, tel que l'agarose réticulée, commercialisée sous la désignation SEPHAROSE. De toute façon, la présence de quantités résiduelles de virus dans la préparation vaccinante n'est que d'une importance relativement mineure. En effet, l'adénovirus n'a qu'un pouvoir pathogène faible chez l'homme. Il n'entraîne que des infections respiratoires bénignes.

La faible importance du pouvoir pathogène des adénovirus, plus particulièrement de ceux qui appartiennent au groupe C des adénovirus humains, permet d'envisager la constitution de "vaccins vivants". Ceux-ci peuvent être constitués par des adénovirus infectieux modifiés au niveau de la région E3 par l'insertion de l'ADN recombinant selon l'invention dans la partie non essentielle de l'adénovirus. A cet égard, il faut souligner le fait que les adénovirus humains du groupe C ne se sont jamais révélés tumorigènes chez l'animal (3). Ces vecteurs ou virus seront d'un intérêt tout particulier pour la transformation de cellules Vero, dont le caractère non tumorigène est maintenant solidement établi. De ce fait elles constituent une lignée d'origine animale particulièrement favorable à la production de produits à usage humain.

Des caractéristiques supplémentaires de l'invention apparaîtront encore au cours de la description qui suit de constructions préférées de vecteurs contenant l'ADN recombinant selon l'invention et des conditions dans lesquelles ces vecteurs sont utilisables. Il sera fait référence à cette occasion aux dessins dans lesquels :

- la fig. 1 représente schématiquement les positions relatives des différentes régions du génome d'un adénovirus du sous-type Ad5, d'une part, et à échelle agrandie, de la région E1A de ce génome ;
- la fig. 2 est un schéma désormais classique du génome du virus de l'hépatite B ;
- les fig. 3 à 6 montrent les constructions successives qui ont conduit à la réalisation d'un plasmide contenant un premier ADN recombinant conforme à l'invention (fig. 6) et
- la fig. 7 représente schématiquement la construction d'un "virus recombinant défectif" à partir du plasmide modifié de la fig. 5 et de génomes défectifs de Ad5.

On fera d'abord les observations suivantes à propos des figures, avant de décrire la réalisation de constructions d'ADNs recombinants selon l'invention.

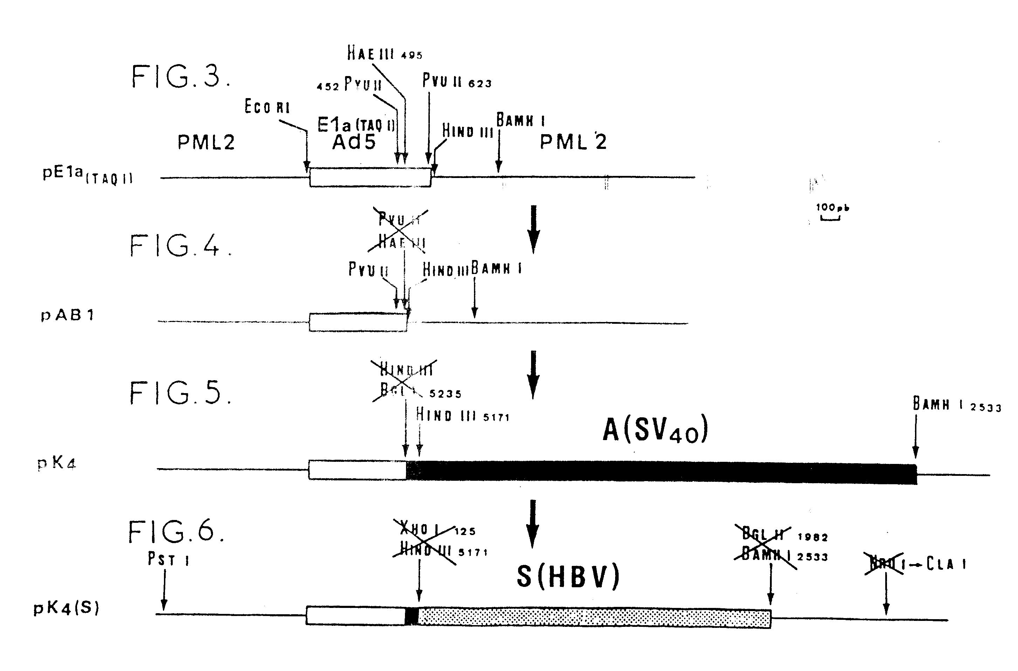

Dans les fig. 3 à 6, les parties en trait fin correspondent à des séquences du plasmide pML2.

Les nombres apparaissant dans les fig. 1 à 5 indiquent les positions des sites de restriction dans les séquences virales Ad5, SV40 et HBV. Les numérotations des positions des sites de restriction de Ad5 et du SV450 sont celles de J. TOOZE (1), celles de l'HBV sont celles de P. TIOLLAIS et al. (11).

Les raturations des désignations de sites dans les dessins témoignent de la présence antérieure des mêmes sites dans les parties correspondantes des ADNs qui seront décrits ci-après. Ces sites ont cependant été délétés, supprimés par réparation des extrémités cohésives des fragments ouverts ou fragmentés à l'aide des enzymes de restriction correspondasntes, ou par tout autre moyen tel qu'envisagé dans la description qui suit des constructions qui ont été faites.

1. Rappel des principaux éléments de la structure et de l'organisation du génome de l'adénovirus Ad5 (ci-après souvent simplement désigné par la désignation Ad5) :

Ils résultent des parties 1A et 1B de la fig. 1.

1A : Le génome est une molécule de DNA linéaire double brin longue d'environ 36.000 pb. Les flèches indiquent la position et le sens des transcriptions des régions précoces E1a, E1b, E2, E3 et E4. Le promoteur majeur tardif $PM_t$ et l'unité de transcription qui lui est associée sont également montrés. Les numérotations de 10 en 10, de 0 à 100, correspondent à des tailles exprimées en % de la taille du génome total.

1B : La région E1A. Les transcripts de cette région ont tous des extrémités 5'P (position 499) et 3'OH (position 1632) identiques. Le premier T de l'élément TATA du promoteur est situé à la position 468. Les sites de restriction utilisés dans les constrructions des plasmides sont indiqués. Les tailles sont exprimées par des nombres de paires de bases.

6

2. Origine des fragments contenant le gène S ou le gène S et la région pré-S2 utilisés dans les constructions qui suivent.

1° cas : Fragment du génome du virus de l'hépatite B contenant le gène S.

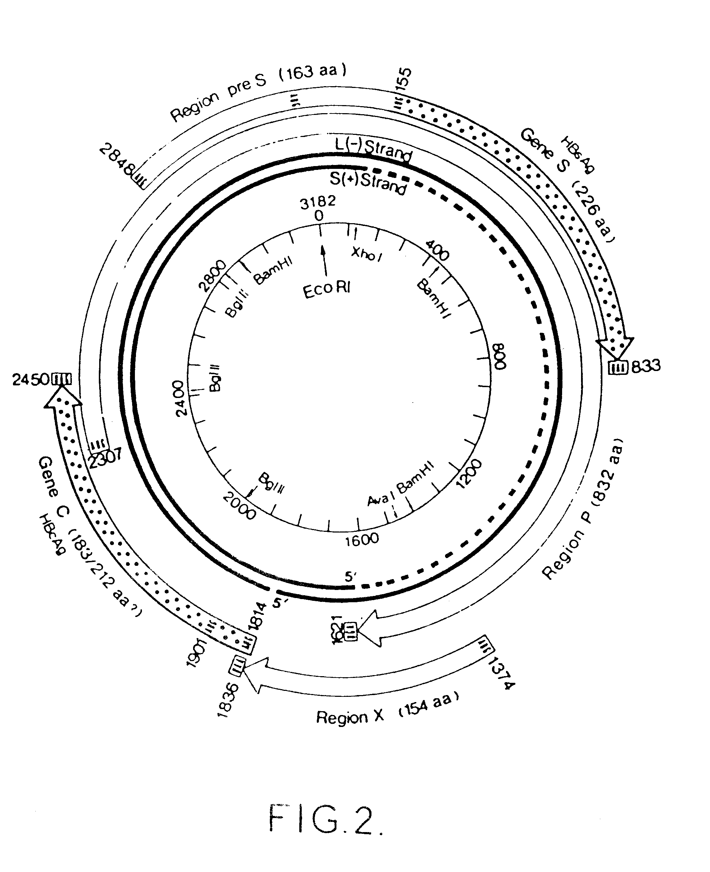

Il est issu du génome du virus de l'hépatite B (fig. 2). Il est rappelé que le génome du virus de l'hépatite B est une molécule de DNA circulaire partiellement simple brin. Sa longueur est d'environ 3.200 pb. Il est constitué par l'appariement de deux brins de longueur inégale appelés brins L(-) et S(+). Le gène S représente la séquence codante du polypeptide majeur de l'enveloppe virale qui porte l'HBsAg. Le fragment d'ADN utilisé dans les constructions ci-après est le fragment $XhoI_{127}$-$BglIII_{1984}$. Le site de polyadénylation du messager de l'HBsAg a été localisé à la position 1916.

2° cas : Fragment du génome du virus de l'hépatite B contenant le gène S et la région pré-S2.

Le fragment d'ADN utilisé est le fragment $MstII_{3161}$-$BglIII_{1982}$ qui code à la fois pour l'antigène HBs et pour un récepteur de la sérumalbumine humaine polymérisée (pHSA). Le site MstII précède le codon d'initiation de la région pré-S2 de 9 nucléotides. Le site BglII est situé à 64 nucléotides en aval du signal de poly A addition du gène S (de l'anglais "poly A addition signal").

Il est question dans ce qui suit de la construction et de la propagation d'un adénovirus recombinant comportant le gène S. Le mode opératoire est identique pour obtenir un adénovirus recombinant, conforme à l'invention, possédant le gène S et la région pré-S2, étant entendu que, dans ce second cas, c'est le fragment d'ADN MstII-BglIII qui est inséré entre les sites de restriction HindIII et BamHI du plasmide pK4, à la place du fragment d'ADN XhoI-BglIII dont il est question ci-après.

On désignera dans ce qui suit par Ad5(X-B) l'adénovirus recombinant possédant le gène S et par Ad5-(M-B) l'adénovirus recombinant possédant le gène S et la région pré-S2.

3. Construction du plasmide pE1A(TaqI) (fig. 3).

Le plasmide pE1A(TaqI) contient les 632 premiers nucléotides de l'extrémité gauche du génomé de l'Ad5. Ce fragment a été obtenu par coupure du fragment de restriction purifié SacI E (0 - 5,0 %) de l'Ad5 par TaqI (fig. 1). Ce fragment a été inséré entre les sites de restriction EcoRI et ClaI du plasmide pML2 (fig. 3) Le plasmide pML2 a été ouvert par EcoRI et ClaI. Le fragment de Ad5 a été lié au plasmide linéarisé au niveau de l'extrémité TaqI. La jonction des extrémités TaqI-ClaI recrée un site de restriction ClaI. L'extrémité EcoRI du recombinant a été réparée avec la DNA-polymérase I de E. coli (fragment de Klenow) et le plasmide recircularisé au moyen de la ligase T4. Le site EcoRI a donc été reconstitue.

4. Fabrication du plasmide pAB1 (fig. 4).

Le plasmide pAB1 a été construit à partir du plasmide pEIA(TaqI), de façon à éliminer la partie codante de la région E1A. Ceci a été effectué par isolement du fragment PvuII-PvuII (positions 452-623), coupure de ce fragment par l'enzyme HaeIII (position 495), réinsertion du fragment $PvuII_{452}$-$HaeIII_{495}$ au niveau du site $PvuII_{623}$ du plasmide pE1A(TaqI). En d'autres termes, le fragment $HaeIII_{495}$-$Pvu_{623}$ a été délété. Le plasmide pAB1 contient un site HindIII proche du site d'initiation de transcription de la région E1A et un site BamHI (de pML2) situé à distance. Ces deux sites de restriction peuvent être utilisés pour cloner des genes étrangers sans qu'il y ait fusion génétique.

5. Production du plasmide pK4 (fig. 5).

pAB1 a été coupé par HindIII et BamHI et le fragment contenant le promoteur de E1A, issu de pAB1, a été lié, au niveau de son extrémité BamHI au fragment BglI-BamHI (positions 5235-2533 du génome du virus SV40) ci-après dénommé A(SV40) contenant le gène codant pour les antigènes T et t du virus SV40. Après réparation des extrémités BglI et HindIII du reconstituant par le fragment de Kleenow, le plasmide est recircularisé au moyen de la ligase T4. La construction présente dans le plasmide pK4 a été testée en mettant en jeu l'expression transitoire du gène T. Introduit dans des cellules HeLa par transfection selon la technique au phosphate de calcium (19), le plasmide pK4 dirige la synthèse de l'antigène T de SV40 qui a été détectée par immunofluorescence. Environ 1 % des cellules transfectées présentaient une fluorescence nette. L'absence de fluorescence après transfection cellulaire par un plasmide contenant le fragment de SV40 inséré dans la mauvaise orientation montre que le gène des antigènes T et t est bien placé sous le contrôle du promoteur E1A de l'Ad5.

C'est le site HindIII, à la position 5171 du fragment A(SV40) qui est ensuite utilisé pour substituer le susdit fragment contenant le gène 5 à la majeure partie de A(SV40).

### 6. Production du plasmide pK4S (X-B)(fig. 6).

Le plasmide pK4 a été digéré par HindIII et BamHI. Le fragment XhoI-BglII (positions 125 à 1982) du génome du virus de l'hépatite B (HBV) (fig. 2) a été inséré, en lieu et place de la majeure partie de A(SV40) entre les sites de restriction HindIII et BamHI du plasmide pK4, après réparation de leurs extrémités respectives par la DNA polymérase I d'E. coli (fragment de Kleenow) (fig. 3). Les sites de restriction XhoI, HindIII, BamHI et BglII sont perdus après ligature.

Le site HindIII était situé à 8 nucléotides en amont de l'ATG initiateur des antigenes T et t. L'insertion du gène S dans ce site permet donc de conserver l'extrémité 5 du mRNA précoce de SV40 contenant le site de "capping" et les séquences d'appariement du messager aux ribosomes. Le fragment de DNA HBV contient la séquence codante ou gène S (position 155 à 833) du polypeptide majeur de l'enveloppe virale porteur de l'HBsAg ainsi que la séquence située en 3' du gène S et qui inclut le site de polyadénylation de l'ARN messager de l'HBsAg à la position 1916 (20, 21, 22). Deux plasmides $pK4S^+$ et $pK4S^-$ porteurs du fragment HBV inséré dans les deux sens ont été isolés. Des cellules 293 ont été transfectées par ces deux plasmides et la synthèse d'HBsAg a été recherchée dans le surnageant cellulaire 3 jours après la transfection. Seul le plasmide $pK4S^+$ qui possède le promoteur E1A à l'extrémité 5' du gène S est capable de diriger la synthèse d'HBsAg. Ceci montre que l'expression du gène S est bien sous le contrôle du promoteur E1A de l'Ad5. Finalement, un site de restriction ClaI non méthylé dans E. coli a été introduit dans le plasmide $pK4S^+$ (X-B) au niveau du site NruI de la séquence $pML_2$. Le site est nécessaire à la construction du virus recombinant.

C'est finalement le fragment délimité par des extrémités PstI et ClaI, et obtenu à partir de $pK4S^+$ (X-B), qui a été utilisé pour la fabrication d'un "virus recombinant défectif" conforme à l'invention.

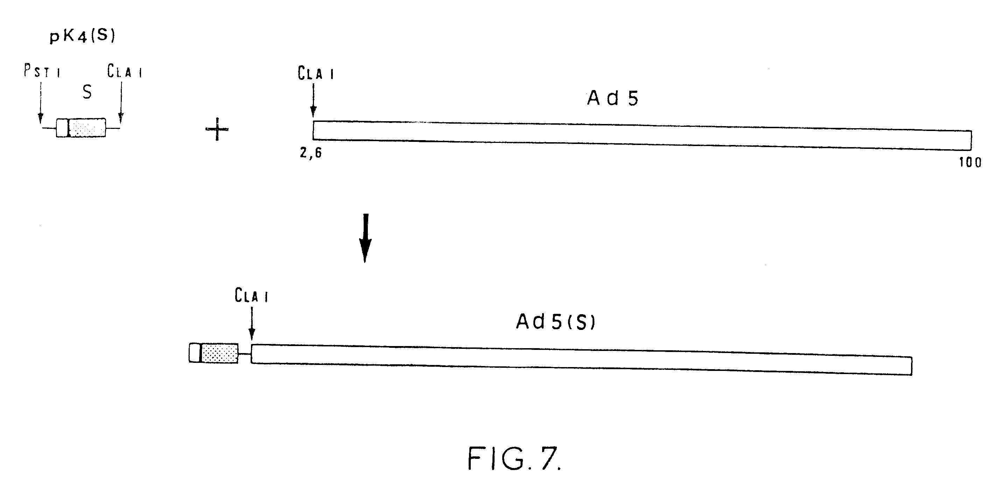

### 7. Construction du virus recombinant défectif (fig. 7).

3 microgrammes du fragment de restriction Pst1-ClaI purifié à partir du plasmide $pK4S^+$ (X-B) ont été ligaturés à 20 microgrammes du fragment de restriction ClaI (2,6 % - 100 %) de l'Ad5 purifié par ultracentrifugation en gradient de saccharose pour fournir le "virus défectif recombinant" Ad5.

### 8. Pronagation du virus recombinant.

Les cellules 293 ont été cultivées dans des boîtes de 6 cm de diamètre. 4 heures avant la transfection le surnageant de culture a été remplacé par du milieu. 5 boîtes de cellules 293 à 70 % de confluence ont alors été transfectées avec le mélange de ligation selon la technique au phosphate de calcium puis incubées pendant 4 heures à 37°C. Après adsorption, les cellules contenues dans chaque boîte ont été lavées avec 2 ml de tampon TS (NaCl 8000,0 mg/l, KCl 380,0 mg/l, $Na_2$ $HPO_4$ 100,0 mg/l, $CaCl_2$ 100,0 mg/l, $MgCl_2$, $6H_2O$ 100,0 mg/l, Tris 3000,0 mg/l pH 7,4), traitées avec 400 microlitres d'une solution TS contenant 20 % de glycérol pendant 1 minute à température ordinaire, lavées deux fois avec 2 ml de tampon TS, puis recouverte avec 4 ml de milieu MEM contenant 1 % d'agar noble, 1 % de sérum de veau foetal. Aux jours 4 et 7, les cellules ont été recouvertes avec 4 ml du mélange nutritif. Aujour 10, les cellules ont été colorées avec 4 ml du milieu nutritif supplémenté avec 0,01 % de rouge neutre. Les plages ont été observées au jour 11. Les virus ont été resuspendus dans 1 ml de TS et amplifié sur cellules 293. La présence de HBsAG dans le milieu de culture a été testée par RIA (Austria II, laboratoire ABBOTT). Après amplification, la présence de séquences HBV dans le recombinant a été testée par hybridation. Cinq plages ont été analysées. Une seule contrenait un virus recombinant $HBsAg^+$. Ce clone Ad5(X-B) ainsi qu'un autre clone étaient positifs pour la détection de séquences HBV. La taille du génome viral recombinant excède celle du virus sauvage de 2100 pb. Aucune délétion n'a pu être détectée par analyse de fragments de restriction du génome recombinant. Par ailleurs, cette analyse a montré que les séquences du pML2 situées entre le site de restriction PstI et la séquence d'Ad5 ont été correctement excisées au cours de la propagation du génome recombinant dans la lignée 293.

### 9. La synthèse d'HBsAg dirigée par le vecteur Ad5(X-B).

Des cellules 293 et des cellules Vero ont été infectées par le virus Ad5(X-B). Les niveaux d'expression de HBsAg synthétisé sont montrés dans le tableau I. Des échantillons du surnageant cellulaire ont été prélevés 3 jours après l'infection et l'HBsAg a été recherché par essai radioimmunologique ("radio-immunoassay : RIA). Les résultats montrent que le vecteur Ad5(X-B) est capable de diriger la synthèse d'HBsAg dans ces deux lignées cellulaires, et l'excrétion d'HBsAg par les lignées cellulaires dans leurs milieux de culture respectifs.

L'HBsAg synthétisé a été purifié par ultracentrifugation en CsCl. Il a une densité de 1,20. Des particules typiques de 22 nm ont été observées par microscopie électronique.

### 10. Synthèse d'HBsAg dirigée par les vecteurs Ad5(M-B).

Les niveaux d'expression de l'HBsAg synthétisé après l'infection de cellules 293 et de cellules Vero par Ad5(M-B) sont montrés dans le tableau I.

La cinétique de la distribution extra- et intracellulaire de HBsAg à partir de cellules Vero infectées par Ad5(M-B) a indiqué que la synthèse d'HBsAg a commencé 3 heures après l'infection et a pu être détectée dans le milieu après 8 heures. L'infection par le virus recombinant Ad5(M-B) a conduit à une accumulation de HBsAg de 0,5 à 1 $\mu g/10^6$ cellules dans le milieu après 120 heures. Des expériences répétées ont montré que le virus recombinant contenant la région pré-S2 synthétise de plus grandes quantités de HBsAg que le virus recombinant ne contenant que le gène S. L'HBsAg purifiée à partir du milieu de culture de cellules infectées par l'adénovirus recombinant Ad5(M-B) a consisté en une population homogène de particules ayant un diamètre moyen de 22 nm. La densité après centrifugation en CsCl a été de 1,21.

### 11. Activité de récepteur pour la pHSA des particules HBsAq issues de cellules Vero.

Les particules HBsAg produites dans les cellules Vero ont été testées par la technique d'hémagglutination de globules rouges de moutons recouverts de pHSA et par essai radioimmunologique (RIA) pour déceler la présence d'une activité de récepteur pour la pHSA. Une activité de fixation pour la pHSA a été détectée, mais pas pour l'albumine bovine polymérisée (tableau II). Une telle activité n'a pas été décelée avec les cellules Vero infectées par l'adénovirus recombinant Ad5(X-B) contenant seulement le gène S.

### 12. Activité in vivo du virus recombinant.

Des lapins ont été inoculés intraveineusement avec des préparations hautement purifiées de l'adénovirus recombinant Ad5(M-B) et de l'adénovirus sauvage. Bien que l'antigène HBsAg n'ait pu être détecté dans leur sérum, 5 lapins sur 8, inoculés avec le virus recombinant, ont montré l'apparition d'un titre anti-HBs variant de 20 à 270 mIU/ml après 15 jours (tableau III). Aucun anticorps anti-HBs n'a été détecté dans les lapins auxquels on a injecté le type sauvage d'adénovirus. Après une seconde inoculation intraveineuse réalisée 4 semaines après la première, un second pic a été observé dans la réponse anti-HBs atteignant 440 mIU/ml pour l'un des animaux. 4 semaines après la seconde injection, des titres d'anti-HBs variant de 6 mIU/ml à 360 mIU/ml étaient relevés. Des études antérieures ont indiqué que le niveau minimum d'anti-HBs encore protecteur contre l'HBV est de 10 mIU/ml pour l'homme. Les anticorps anti-pHSA ont été recherchés chez les lapins inoculés dans le but de déterminer leur rapport avec la neutralisation de l'HBV. Ces anticorps, détectés par l'inhibition de l'hémagglutination, ont été trouvés dans 5 animaux sur 5 ayant une réponse anti-HBs positive (tableau IV).

L'adénovirus recombinant Ad5(M-B) dirige donc in vivo la synthèse de particules HBsAg ayant un caractère récepteur pour la sérumalbumine humaine polymérisée.

L'invention fournit donc une base méthodologique pour la fabrication d'un vaccin contre l'hépatite B (ou contre d'autres types d'affections) dans des cultures cellulaires.

L'intérêt de l'utilisation de l'adénovirus de type 5 comme vecteur est double. D'une part, ce virus est un virus dont le pouvoir pathogène chez l'homme est faible. Il n'entraîne que des infections respiratoires bénignes. D'autre part, ce sérotype qui appartient au groupe C des adénovirus humains n'est pas tumorigène chez l'animal. D'autre part, le taux de production d'HBsAg obtenu sur cellules Vero (environ 1 microgramme/$10^6$/par cycle infectieux) est a priori suffisant pour une exploitation industrielle. Or cette lignée cellulaire est non tumorigène et pour cette raison, elle constitue la lignée d'origine animale a priori la plus favorable à la production d'un produit à usage humain.

Il va également de soi que peuvent être substitués aux cellules 293 qui ont été mentionnées plus haut toutes autres cellules d'eucaryotes supérieurs infectables par des adénovirus ou susceptibles de reconnaître le promoteur E1A des adénovirus, ces cellules ayant été modifiées, par incorporation au préalable dans leurs propres génomes, d'une séquence contenant les parties manquantes au virus recombinant défectif selon l'invention du génome d'un adénovirus, notamment sous le contrôile d'un promoteur fort reconnu par ces cellules, par exemple d'un promoteur de la thymidine kinase ou d'un promoteur de virus SV40. La séquence originaire de l'adénovirus, alors intégrée dans le génome de ces cellules d'eucaryotes supérieurs, pourra donc complémenter les virus défectifs conformes à l'invention, dans des conditions analogues à celles permises par les cellules 293. Ces méthodes sont applicables avec un avantage particulier à des cellules Vero.

L'adénovirus Ad5 mis en oeuvre a été déposé le 3 août 1984 sous le n° I-322 à la C.N.C.M. ("Collection Nationale de Cultures de Micro-organismes de l'INSTITUT PASTEUR de Paris.

La lignée 293 a été déposée à la C.N .C.M. le 3 août 1984 sous le n° I-323.

TABLEAU I

| Production de HBsAg extracellulaire en fonction du temps | | | |
|---|---|---|---|
| Jour | Ad5(X-B) Cellules Vero infectées | Ad5(X-B) Cellules 293 infectées | Ad5(M-B) Cellules Vero infectées | Ad5(M-B) Cellules 293 infectées |
| 1 | 13 | 15 | 106 | 35 |
| 2 | 17 | 90 | N.D. | N.D. |
| 3 | 45 | 110 | 349 | 56 |
| 4 | 110 | 100 | 668 | 60 |
| 5 | 320 | 130 | 1 057 | 63 |
| 6 | 470 | 120 | 1 153 | N.D. |
| 7 | 550 | 120 | 900 | N.D. |

Les quantités cumulatives de HBsAg (en ng) produites après infection de cellules 293 et de cellules Vero, soit par Ad5(X-B), soit par Ad5(M-B) sont indiquées.

## TABLEAU II

### Activité de récepteur pour la pHSA des particules HBsAg à partir de cellules Vero.

| | Surnageant de culture de cellules | | | Sérum humain | |
|---|---|---|---|---|---|
| | Ad5(X-B) | Ad5(M-B) | Témoin non infecté | HBsAg+ HBeAg+ | Témoin sain |
| | RIA  HA | RIA  HA | RIA  HA | RIA  HA | RIA  HA |
| pHSA | 143  – | 7 512 64 | 119  – | 10 472 128 | 65  – |
| pBSA | 58  ND | 85 ND | 77  ND | 145  ND | 81  ND |

Les résultats sont exprimés en cpm pour l'essai radioimmunologique en phase solide et en titre d'hémagglutination (HA).

ND, non déterminé.

pHSA : sérumalbumine humaine polymérisée.

pHSA : sérumalbumine bovine polymérisée.

cpm : (counts per minute).

TABLEAU III

| Réponse anti-HBs (mIU ml$^{-1}$) chez les lapins inoculés. | | | | | | | | | | |
|---|---|---|---|---|---|---|---|---|---|---|
| Semaines | Lapins | | | | | | | | | |
| | 1 | 2 | 3 | 4 | 5 | 6 | 7 | 8 | 9 | 10 |
| 0 | 0 | 0 | 0 | 0 | 0 | 0 | 0 | 0 | 0 | 0 |
| 1 | 0 | 0 | 2 | 0 | 0 | 30 | 8 | 6 | 12 | 20 |
| 2 | 2 | 0 | 2 | 0 | 2 | 15 | 20 | 270 | 70 | 85 |
| 3 | 0 | 2 | 0 | 0 | 5 | 18 | 13 | 48 | 15 | 11 |
| 4 | 1 | 0 | 0 | 0 | 2 | 21 | 15 | 27 | 17 | 7 |
| 5 | 0 | 0 | 2 | 0 | 2 | 99 | 52 | 440 | 146 | 136 |
| 6 | 0 | 0 | 0 | 0 | 0 | 78 | 26 | 401 | 151 | 148 |
| 7 | 0 | 0 | 0 | 0 | 0 | 20 | 27 | 325 | 58 | 42 |
| 8 | 0 | 0 | 0 | 0 | 0 | 20 | 40 | 367 | 35 | 6 |

On a fait une injection intraveineuse de $10^9$ pfu de l'adénovirus Ad5 sauvage purifié à des lapins (lapins 1 et 2) et $10^9$ pfu d'adénovirus recombinant Ad5(M-B) purifié à des lapins (lapins 3 à 10) immédiatement après le prélèvement du sang à la semaine 0 et à la semaine 4. La quantité d'anticorps anti-HBs a été mesurée en utilisant le système RIA AUSAB de Abbott et exprimée en unités internationales (3,5 RIA équivalant à 1 mIU). (pfu = plage formant unité).

TABLEAU IV

| Réponse immunogène du recepteur anti-pHSA (réciproque du titre). | | | | | |
|---|---|---|---|---|---|
| Semaines | Lapins | | | | |
| | 6 | 7 | 8 | 9 | 10 |
| 0 | 0 | 0 | 0 | 0 | 0 |
| 1 | 16 | 2 | 2 | 2 | 2 |
| 2 | 4 | 16 | 16 | 16 | 16 |
| 3 | 4 | 4 | 16 | 8 | 8 |
| 4 | 2 | 2 | 4 | 4 | 4 |
| 5 | 16 | 32 | 32 | 32 | 32 |
| 6 | 16 | 32 | 32 | 16 | 32 |
| 7 | 8 | 8 | 32 | 8 | 16 |
| 8 | 4 | 4 | 8 | 4 | 8 |

On a infecté, d'une manière intraveineuse, des lapins avec $10^9$ pfu d'adénovirus recombinant Ad5(M-B) purifiés aux semaines 0 et 4. Les animaux ont été identifiés par les mêmes numéros que dans le tableau III.

L'activité de récepteur anti-pHSA a été exprimée comme la réciproque du titre de sérum le plus élevé capable de fournir une inhibition à 100 % de l'hémagglutination. Les particules d'HBsAg possédant un titre d'hémagglutination récepteur pour la pHSA de 1:128 ont été mélangées avec un volume égal de dilutions en séries de sérums inhibiteurs.

REFERENCES

(1) Edited by Tooze, DNA Tumor Viruses (part II), Cold Spring Harbor Laboratory, 1980.

(2) Berkner, K. and Sharp, P. (1983) Nucleic Acids Res. 11, 6003-6020.

(3) Jones, N. and Shenk, T. (1978) Cell 13, 181-188.

(4) Solnick, D. (1983) The Embo Journal 2, 845-851.

(5) Thummel, C., Tjian, R. and Grodzicker, T. (1981) Cell 23, 825-836.

(6) Berkner, K. and Sharp, P. (1984) Nucleic Acids Res. 12, 1925-1941.

(7) Thummel, C., Tjian, R., Shiu-Lok Hu and Grodzicker, T. (1983) Cell 33, 455-464.

(8) Spector, D., Mc Grogan, M. and Raskas, H. (1978) J. Mol. Biol. 126, 395-414.

(9) Shan, A. R. and Ziff, E. B. (1980) Cell 22, 905-916.

(10) Graham, F.L., Smiley, J., Russel, W.C. and Nairn, R. (1977) J. Gen. Virol. 36, 59-72.

(11) Tiollais, P., Charnay, P. and Vyas, G. (1981) Science 213, 406-411.

(12) Peterson, D.L. (1981) J. Biol. Chem. 256, 6975-6983.

(13) Lusky, M. and Botchan, M. (1981) Nature 293, 79-81.

(14) Enns, R., Challberg, M., Ahern, K., Chow, K., Mathews, C., Astell, R. and Pearson, G. (1983) Gene, 23, 307-313.

(15) Tibbetts, C. (1977) Cell 12, 243-249.

(16) Hammarskjùld, M.L. and Winberg, G. (1980) Cell 20, 787-795.

(17) Stow, N. (1981) J. Virol. 37, 171-180.

(18) D'Halluin, J.C., Milleville, M. and Boulanger, P. (1983) Gene 21, 165-169.

(19) Graham, F.L. and Van Der Eb, A.J. (1973) Virology 52, 456-467.

(20) Charnay, P., Mandart, E., Hampe, A., Fitoussi, F., Tiollais, P., and Galibert, F. (1979) Nucl. Acids Res. 7, 335-346.

(21) Valenzuela, P., Gray, P., Quiroga, M., Zaldivar, J., Goodman, H.M., Rotter, W.J. (1979) Nature 280, 815-819.

(22) Pourcel, C., Louise, A., Gervais, M., Chensiner, N., Dubois, M.F., Tiollais, P. (1982), J. Virol. 42, 100-105.

(23) Stenlund, A., Lamy, D., Moreno-Lopez, S., Ahola, H., Petterson, U. and Tiollais, P. (1982) EMBO J2, 669-673.

(24) Moriarty, A.M., Hoyer, B.H., Shih, J.W.K., Guérin, J.L. and Hamer, D.H. (1981) Proc. Natl. Acad. Sci. 78, 2606-2610.

(25) Heermann, K.H., Goldmann, V., Schwartz, W., Seyffarth, T., Baumgarten, H., et Gerlich, W.H. J. Virol. 52, 396-402, 1984.

(26) Standring, D.N., Rutter, W.J., Varmus, H.E., et Ganem, D. J. Virol. 50, 563-571, 1984.

**Revendications**

1. Vecteur recombinant contenant le promoteur précoce de la région E1A du génome d'un adénovirus pour la transformation de lignées cellulaires eucaryotes, notamment humaines ou animales, choisies parmi celles qui sont infectables par des adénovirus ou dont les polymérases endogènes sont susceptibles de reconnaître les promoteurs des adénovirus, ce vecteur étant en outre modifié par un acide nucléique d'insertion contenant une séquence nucléotidique codant pour une séquence polypeptidique dont l'expression dans lesdites lignées cellulaires est recherchée, caractérisé en ce que ladite séquence d'insertion est substituée aux gènes E1A normalement placés sous le contrôle direct dudit promoteur précoce de la région E1A, cette séquence d'insertion étant elle-même placée sous le contrôle direct de ce promoteur et en ce qu'il comporte, en aval de l'acide nucléique d'insertion, l'ensemble des séquences essentielles d'un génome d'adénovirus qui sont nécessaires à la réplication de l'adénovirus correspondant et qui sont normalement situées en aval des gènes normalement sous le contrôle direct du promoteur précoce E1A dans ledit génome, et en ce que la susdite séquence nucléotidique codant pour la séquence polypeptidique dont l'expression est recherchée est hétérologue vis-à-vis des gènes du susdit adénovirus.

2. Vecteur recombinant selon la revendication 1, caractérisé en ce que le génome de l'adénovirus avec lequel il est associé est défectif, en ce qu'il est dépourvu de la partie antérieure de la région E1A du génome viral, notamment de son fragment 0-2,6 %.

3. Vecteur recombinant selon la revendication 1, caractérisé en ce que l'ADN recombinant que forme le promoteur précoce de la région E1A et la séquence nucléique d'insertion est incorporé dans une région non essentielle d'un adénovirus, notamment dans la partie non essentielle de la région E3 de cet adénovirus.

4. Vecteur recombinant selon la revendication 1, caractérisé en ce qu'il consiste en un adénovirus infectieux comportant l'ADN recombinant que forme le promoteur précoce de la région E1A et la séquence nucléique d'insertion, incorporé dans l'une de ses régions non essentielles, notamment dans la partie non essentielle de sa région E3.

5. Vecteur recombinant selon l'une quelconque des revendications 1 à 4, caractérisé en ce que son promoteur est originaire d'un adénovirus de catégorie C, appartenant au sous-type Ad2 ou Ad5.

6. Vecteur recombinant selon l'une quelconque des revendications 1 à 5, caractérisé en ce que la séquence nucléotidique contenue dans le susdit acide nucléique d'insertion code pour une protéine qui, lorsqu'elle est exprimée dans un hôte cellulaire naturel sous le contrôle de son promoteur naturel, est excrétée dans le milieu d'une culture de cet hôte cellulaire naturel.

7. Vecteur recombinant selon l'une quelconque des revendications 1 à 6, caractérisé en ce que la susdite séquence nucléotidique code pour un antigène protecteur contre un agent pathogène déterminé, notamment pour l'antigène HBsAg.

8. Vecteur recombinant selon la revendication 7, caractérisé en ce que ladite séquence nucléique comprend le gène S et la région pré-S2 du génome du virus l'hépatite B.

9. Lignée cellulaire d'origine humaine ou animale, caractérisée en ce qu'elle est transformée par un vecteur recombinant selon l'une quelconque des revendications 1 à 8, et en ce qu'elle synthétise le polypeptide codé par la susdite séquence nucléotidique.

**10.** Lignée cellulaire selon la revendication 9 considérée en combinaison avec la revendication 1 ou la revendication 2, ou les deux à la fois, et, le cas échéant, avec l'une quelconque des revendications 5 à 8, caractérisée en ce qu'elle contient elle-même une séquence distincte de nucléotides apte à complémenter la partie du génome de l'adénovirus dont le susdit vecteur est dépourvu, ladite séquence distincte étant de préférence incorporée au génome des cellules de ladite lignée cellulaire.

**11.** Procédé de production d'un polypeptide déterminé comprenant la transformation par un vecteur recombinant contenant une séquence nucléotidique codant pour ledit polypeptide d'une lignée de cellules eucaryotes, notamment animale ou humaine, choisie parmi celles qui sont infectables par des adénovirus ou dont les polymérases endogènes sont aptes à reconnaître les promoteurs des adénovirus, caractérisé par le fait que l'ADN transformant est conforme à l'une quelconque des revendications 1 à 8 et en ce que l'on recueille les produits d'expression de ces cellules, y inclus ledit polypeptide déterminé.

**12.** Procédé selon la revendication 11, caractérisé en ce que le vecteur recombinant est conforme à l'une quelconque des revendications 5 à 8.

**13.** Procédé selon la revendication 11, caractérisé par le fait que les cellules eucaryotes transformées sont conformes à la revendication 10.

**14.** Procédé selon la revendication 11, caractérisé par le fait que les susdites cellules eucaryotes sont simultanément transformées avec l'adénovirus infectieux correspondant.

**15.** Procédé selon l'une quelconque des revendications 11 à 14, caractérisé en ce que la séquence nucléotidique déterminée est conforme à celle définie dans la revendication 7.

**16.** Procédé de production d'un polypeptide possédant des propriétés immunogènes caractéristiques du virus de l'hépatite B comprenant la transformation d'une lignée de cellules eucaryotes, notamment animales ou humaines, choisie parmi celles qui sont infectables par des adénovirus ou dont les polymérases endogènes sont aptes à reconnaître les promoteurs des adénovirus, par un vecteur recombinant contenant un insérat comprenant lui-même une séquence nucléotidique codant pour un polypeptide immunogène caractéristique du virus de l'hépatite B, caractérisé en ce que la susdite séquence nucléotidique est substituée aux gènes E1A normalement placés sous le contrôle direct du promoteur précoce de la région E1A du génome d'un adénovirus, cette séquence d'insertion étant elle-même placée sous le contrôle direct de ce promoteur et ce vecteur recombinant contenant en outre, en aval de l'acide nucléique d'insertion, l'ensemble des séquences d'un génome d'adénovirus qui sont essentielles nécessaires à la réplication de l'adénovirus correspondant et qui sont normalement situées en aval des gènes normalement sous le contrôle direct du promoteur précoce E1A dans ledit génome.

**17.** Procédé selon la revendication 16, caractérisé en ce que le vecteur recombinant consiste en un adénovirus défectif réplicable dans lesdites lignées de cellules, ledit adénovirus étant dépourvu de la partie antérieure de la région E1A du génome viral, notamment de son fragment 0-2,6 %, et en ce qu'on recueille dans le milieu de culture les produits d'expression des lignées de cellules eucaryotes transformées par l'ADN recombinant, y inclus le polypeptide codé par la susdite séquence nucléotidique et synthétisé par lesdites lignées de cellules.

**18.** Procédé selon les revendications 16 ou 17, caractérisé en ce que le promoteur de l'ADN recombinant est originaire d'un adénovirus de catégorie C, appartenant au sous-type Ad2 ou Ad5.

**19.** Procédé selon l'une quelconque des revendications 16 à 18, caractérisé en ce que l'ADN recombinant que forme le susdit promoteur précoce et la séquence d'insertion est incorporé dans une région non essentielle de l'adénovirus, notamment dans la partie non essentielle de la région E3 de cet adénovirus.

**20.** Procédé selon l'une quelconque des revendications 16 à 19, caractérisé en ce que la lignée de cellules eucaryotes contient elle-même une séquence distincte de nucléotides aptes à complémenter la partie du génome de l'adénovirus dont le susdit vecteur est dépourvu, ladite séquence étant de préférence incorporée au génome des cellules de ladite lignée de cellules.

**21.** Procédé selon l'une quelconque des revendications 16 à 20, caractérisé en ce que l'ADN recombinant comprend la séquence nucléotidique correspondant au gène S du génome du virus de l'hépatite B.

**22.** Procédé selon l'une quelconque des revendications 16 à 20, caractérisé en ce que l'ADN recombinant comprend la séquence correspondant au gène S et à la région pré-S2 du génome du virus de l'hépatite B.

**23.** Vaccin dont le principe actif consiste en un adénovirus infectieux et réplicable comportant, incorporé dans l'une de ses régions non essentielles, notamment la partie non essentielle de sa région E3, un ADN recombinant formé entre le promoteur précoce de la région E1A et une séquence nucléique d'insertion codant pour un antigène protecteur contre l'agent pathogène choisi, et notamment contre le virus de l'hépatite B, cette séquence nucléique d'insertion étant substituée aux gènes E1A normalement sous le contrôle de ce promoteur.

**Claims**

**1.** Recombinant vector containing the early promoter of the E1A region of the genome of an adenovirus for the transformation of eucaryotic, particularly human or animal cell lines, selected among those which are infectable by adenoviruses or whose endogenous polymerases are liable of recognizing the promoters of adenoviruses, said vector being further modified by a nucleic acid insertion containing a nucleotide sequence coding for a polypeptide sequence whose expression in said eucaryotic cell lines is sought, characterized in that said insertion sequence is substituted for the E1A genes normally placed under the direct control of said early promoter of the E1A region, said insertion sequence being itself placed under the direct control of said promoter, and in that it comprises, downstream of said nucleic acid insertion, all of the essential sequences of an adenovirus genome which are necessary for the replication of the corresponding adenovirus and which are normally located downstream of the genes normally under the direct control of the E1A early promoter in said genome, and in that said nucleotide sequence coding for the polypeptide sequence whose expression is sought is heterologous with respect to the genes of said adenovirus.

**2.** Recombinant vector according to claim 1, characterized in that the genome of the adenovirus with which it is associated is defective, in that it is deprived of the anterior part of the E1A region of the viral genome, particularly of its 0-2,6 % fragment.

**3.** Recombinant vector according to claim 1, characterized in that the recombinant DNA formed by said early promoter of the E1A region and the insertion nucleic sequence is incorporated in the non-essential region of an adenovirus, particularly in the non-essential part of the E3 region of said adenovirus.

**4.** Recombinant vector according to claim 1, characterized in that it consists of an infectious adenovirus comprising the recombinant DNA formed by the early promoter of the E1A region and the nucleic sequence insertion, incorporated in one of its non-essential regions, particularly in the non-essential part of the E3 region.

**5.** Recombinant vector according to any one of claims 1 to 4, characterized in that its promoter originates from an adenovirus of category C, belonging to sub-type Ad2 or Ad5.

**6.** Recombinant vector according to any one of claims 1 to 5, characterized in that the nucleotide sequence contained in the aforesaid nucleic acid insertion codes for a protein which, when it is expressed in a natural cellular host under the control of its natural promoter, is excreted into the culture medium of this natural cellular host.

**7.** Recombinant vector according to any one of claims 1 to 6, characterized in that the aforesaid nucleotide sequence codes for a protective antigen against a given pathogenic agent, particularly for the HBsAg antigen.

**8.** Recombinant vector according to claim 7, characterized in that said nucleic acid sequence comprises the S gene and the pre-S2 region of the genome of hepatitis B.

9. A cell line of human or animal origin, characterized in that it is transformed by a recombinant vector according to any one of claims 1 to 8, and in that it synthesizes the polypeptide coded by the aforesaid nucleotide sequence.

10. The cell line according to claim 9 considered in combination with claim 1 or claim 2 or both at once, and, as the case may be, with any one of claims 5 to 8, characterized in that it itself contains a distinct sequence of nucleotides able to complement the part of the genome of the adenovirus which the aforesaid vector is deprived of, the said distinct sequence preferably being incorporated into the genome of the cells of said cell line.

11. A process of production of a determined polypeptide including the transformation by a recombinant vector containing a nucleotide sequence coding for said polypeptide of the cells of a eucaryotic, particularly animal of human cell line, chosen among those which are infectable by the adenoviruses or whose endogenous polymerases are able to recognize the promoters of the adenoviruses, characterized by the fact that the transforming DNA is in accordance with any one of claims 1 to 8 and in that the expression products, including said specified polypeptide of these cells are recovered.

12. The process according to claim 11, characterized in that the recombinant vector is in accordance with any one of claims 5 to 8.

13. The process according to claim 11, characterized by the fact that the aforesaid transformed eucaryotic cells are in accordance with claim 10.

14. The process according to claim 11, characterized by the fact that the aforesaid transformed eucaryotic cells are simultaneously transformed with the corresponding infectious adenovirus.

15. The process according to any one of claims 11 to 14, characterized in that the specific nucleotide sequence is in accordance with that defined in claim 7.

16. A process of production of a polypeptide possessing the immunogenic properties characteristic of the virus of hepatitis B including the transformation of a line of eucaryotic, particularly animal or human cells, chosen from among those which are infectable by adenoviruses of whose endogenous polymerases are able to recognize the adenovirus promoters, by a recombinant vector containing an insert itself containing a nucleotide sequence coding for an immunogenic polypeptide characteristic of the virus of hepatitis B, characterized in that the aforesaid nucleotide sequence is substituted for the E1A genes normally placed under the direct control of the early promoter of the E1A region of the genome of an adenovirus, said insertion sequence being itself placed under the direct control of said promoter and said recombinant vector further containing, downstream of said nucleic acid insertion, all of the sequences of an adenovirus genome which are essential and necessary for the replication of the corresponding adenovirus and which are normally located downstream of the genes normally under the direct control of the early E1A promoter in said genome.

17. The process according to claim 16, characterized in that the recombinant vector consists of a defective andenovirus which can be replicated in said cell lines, said adenovirus being deprived of the anterior part of the E1A region of the viral genome, particularly of its 0-2,6 % fragment, and in that one recovers in the culture medium the expression products of the eucaryotic cell lines transformed by the recombinant DNA, including the polypeptide coded by the aforesaid nucleotide sequence and synthesized by said cell lines.

18. The process according to claims 16 to 17, characterized in that the promoter of the recombinant DNA originates from an adenovirus of category C, belonging to sub-type Ad2 or Ad5.

19. The process according to any one of claims 16 to 18, characterized in that the recombinant DNA formed by said early promoter and said insertion sequence is incorporated into a non-essential region of the adenovirus, particularly into the non-essential part of the E3 region of this adenovirus.

16

**20.** The process according to any one of claims 16 to 19, characterized in that the eucaryotic cell line itself contains a distinct sequence of nucleotides able to complement the part of the genome of the adenovirus of which the aforesaid vector is deprived, the said sequence preferably being incorporated into the genome of the cells of said cell line.

**21.** The process according to any one of claims 16 to 20, characterized in that the recombinant DNA includes a nucleotide sequence corresponding to the S gene of the genome of the virus of hepatitis B.

**22.** The process according to any one of claims 16 to 20, characterized in that the recombinant DNA includes the sequence corresponding to the S gene and the pre-S2 region of the genome of the virus of hepatitis B.

**23.** Vaccine whose active principle consists of an adenovirus which is infectious and can be replicated and which comprises, incorporated into one of its non-essential regions, particularly the non-essential part of its E3 region, a DNA recombinant formed between the early promoter of the E1A region and a nucleic sequence insertion coding for a protective antigen against the pathogenic agent selected, and particularly against the hepatitis B virus, said nucleic sequence insertion being substituted for the E1A genes normally under the control of said promoter.

**Patentansprüche**

**1.** Rekombinanter Vektor, der den frühen Promotor der Region E1A des Genoms eines Adenovirus für die Transformation von eukaryotischen Zellinien enthält, insbesondere menschlichen oder tierischen, ausgewählt unter jenen, die durch Adenoviren infizierbar sind, oder deren endogene Polymerasen fähig sind, die Promotoren der Adenoviren zu erkennen, wobei dieser Vektor außerdem modifiziert ist durch eine Nukleinsäure-Insertion, die eine für eine Polypeptidsequenz kodierende Nukleotidsequenz enthält, deren Expression in den genannten Zellinien angestrebt ist, dadurch gekennzeichnet, daß die genannte Sequenz der Insertion die E1A-Gene, die normalerweise unter die direkte Kontrolle des genannten frühen Promotors der Region E1A gestellt sind, substituiert, wobei diese Sequenz der Insertion ihrerseits unter die direkte Kontrolle dieses Promotors gestellt ist und dadurch, daß sie unterhalb der Nukleinsäure der Insertion die Anordnung der essentiellen Sequenzen eines Adenovirus-Genoms umfaßt, die zur Replikation des entsprechenden Adenovirus notwendig sind und die normalerweise unterhalb der Gene angeordnet sind, die normalerweise unter der direkten Kontrolle des frühen Promotors E1A in dem genannten Genom sind, und dadurch, daß die genannte für die Polypeptidsequenz kodierende Nukleotidsequenz, deren Expression angestrebt wird, gegenüber den Genen des genannten Adenovirus heterolog sind.

**2.** Rekombinanter Vektor nach Anspruch 1, dadurch gekennzeichnet, daß das Genom des Adenovirus, mit dem er verbunden ist, unvollständig ist, dadurch, daß es den vorderen Teil der Region E1A des Virus-Genoms nicht aufweist, insbesondere sein Fragment 0-2,6 %.

**3.** Rekombinanter Vektor nach Anspruch 1, dadurch gekennzeichnet, daß die rekombinante DNA, die durch den frühen Promotor der Region E1A und die Nukleinsequenz der Insertion gebildet ist, in einer nicht essentiellen Region eines Adenovirus inkorporiert ist, insbesondere in den nicht essentiellen Teil der Region E3 dieses Adenovirus.

**4.** Rekombinanter Vektor nach Anspruch 1, dadurch gekennzeichnet, daß er aus einem infektiösen Adenovirus besteht, der die rekombinante DNA umfaßt, die durch den frühen Promotor der Region E1A und die Nukleinsequenz der Insertion gebildet ist, die in einer seiner nicht essentiellen Regionen inkorporiert ist, insbesondere in dem nicht essentiellen Teil seiner Region E3.

**5.** Rekombinanter Vektor nach einem der Ansprüche 1 bis 4, dadurch gekennzeichnet, daß sein Promotor von einem Adenovirus der Kategorie C herstammt, die zum Untertyp Ad2 oder Ad5 gehört.

**6.** Rekombinanter Vektor nach einem der Ansprüche 1 bis 5, dadurch gekennzeichnet, daß die in der genannten Nukleinsäure der Insertion enthaltene Nukleotidsequenz für ein Protein kodiert, das, wenn es in einem natürlichen Zell-Wirt unter der Kontrolle seines natürlichen Promotors exprimiert wird, in das Milieu einer Kultur dieses natürlichen Zell-Wirtes ausgeschieden wird.

**7.** Rekombinanter Vektor nach einem der Ansprüche 1 bis 6, dadurch gekennzeichnet, daß die genannte Nukleotidsequenz für ein Schutzantigen gegen einen bestimmten pathogenen Stoff kodiert, insbesondere für das Antigen HBsAg.

**8.** Rekombinanter Vektor nach Anspruch 7, dadurch gekennzeichnet, daß die genannte Nukleinsequenz das Gen S und die Region pre-S2 des Genoms vom Virus der Hepatitis B umfaßt.

**9.** Zellinie von menschlichem oder tierischem Ursprung, dadurch gekennzeichnet, daß sie durch einen rekombinanten Vektor nach einem der Ansprüche 1 bis 8 transformiert wird und dadurch, daß sie das Polypeptid synthetisiert, das von der genannten Nukleotidsequenz kodiert wird.

**10.** Zellinie nach Anspruch 9, in Kombination mit Anspruch 1 oder 2, oder beiden gleichzeitig, und gegebenenfalls mit einem der Ansprüche 5 bis 8, dadurch gekennzeichnet, daß sie selbst eine bestimmte Nukleotidsequenz enthält, die fähig ist, den Teil des Genoms des Adenovirus zu komplementieren, dem der genannte Vektor fehlt, wobei die genannte bestimmte Sequenz bevorzugt ins Genom der Zellen der genannten Zellinie inkorporiert ist.

**11.** Verfahren zur Herstellung eines bestimmten Polypeptids umfassend die Transformation durch einen rekombinanten Vektor, der eine für das genannte Polypeptid einer eukaryotischen Linie von Zellen kodierende Nukleotidsequenz enthält, insbesondere tierische oder menschliche, ausgewählt von denen, die durch Adenoviren infizierbar sind, oder deren endogene Polymerasen fähig sind, die Promotoren der Adenoviren zu erkennen, dadurch gekennzeichnet, daß die transformierende DNA gemäß einem der Ansprüche 1 bis 8 ist und dadurch, daß die Produkte der Expression dieser Zellen aufgenommen werden, darunter das genannte bestimmte Polypeptid.

**12.** Verfahren nach Anspruch 11, dadurch gekennzeichnet, daß der rekombinante Vektor gemäß einem der Ansprüche 5 bis 8 ist.

**13.** Verfahren nach Anspruch 11, dadurch gekennzeichnet, daß die transformierten eukaryotischen Zellen gemäß Anspruch 10 sind.

**14.** Verfahren nach Anspruch 11, dadurch gekennzeichnet, daß die genannten eukaryotischen Zellen gleichzeitig mit den entsprechenden infektiösen Adenoviren transformiert werden.

**15.** Verfahren nach einem der Ansprüche 11 bis 14, dadurch gekennzeichnet, daß die bestimmte Nukleotidsequenz gemäß der in Anspruch 7 definierten ist.

**16.** Verfahren zur Herstellung eines Polypeptids, das für den Virus der Hepatitis B charakteristische immunogene Eigenschaften besitzt, umfassend die Transformation einer Linie von eukaryotischen Zellen, insbesondere tierischen oder menschlichen, ausgewählt unter jenen, die durch Adenoviren infizierbar sind, oder deren endogene Polymerasen fähig sind, die Promotoren der Adenoviren zu erkennen, durch einen rekombinanten Vektor, der eine Insertion enthält, die selbst eine für ein für den Virus der Hepatitis B charakteristisches immunogenes Polypeptid kodierende Nukleotidsequenz umfaßt, dadurch gekennzeichnet, daß die genannte Nukleotidsequenz die E1A-Gene, die normalerweise unter die direkte Kontrolle des frühen Promotors der Region E1A des Genoms eines Adenovirus gestellt sind, substituiert, wobei diese Sequenz der Insertion selbst unter die direkte Kontrolle dieses Promotors gestellt ist und dieser rekombinante Vektor außerdem, unterhalb der Nukleinsäure der Insertion, die Anordnung der Sequenzen eines Adenovirus-Genoms enthält, die für die Replikation des entsprechenden Adenovirus essentiell notwendig sind und die normalerweise unterhalb der Gene angeordnet sind, die normalerweise unter der direkten Kontrolle des frühen Promotors E1A in dem genannten Genom sind.

**17.** Verfahren nach Anspruch 16, dadurch gekennzeichnet, daß der rekombinante Vektor in einem unvollständigen Adenovirus besteht, der in den genannten Linien von Zellen replizierbar ist, wobei der genannte Adenovirus den vorderen Teil der Region E1A des Virusgenoms nicht aufweist, insbesondere sein Fragment 0-2,6 %, und dadurch, daß im Kulturmilieu die Produkte der Expression der von der rekombinanten DNA transformierten Linien von eukaryotischen Zellen, darin eingeschlossen das von der genannten Nukleotidsequenz kodierte und von den genannten Linien von Zellen synthetisierte Polypeptid aufgenommen werden.

18. Verfahren nach den Ansprüchen 16 oder 17, dadurch gekennzeichnet, daß der Promotor der rekombinanten DNA von einem Adenovirus der Kategorie C herstammt, der zum Untertyp Ad2 oder Ad5 gehört.

19. Verfahren nach einem der Ansprüche 16 bis 18, dadurch gekennzeichnet, daß die rekombinante DNA, die von dem genannten frühen Promotor und der Sequenz der Insertion gebildet ist, in einer nicht essentiellen Region des Adenovirus inkorporiert ist, insbesondere in dem nicht essentiellen Teil der Region E3 dieses Adenovirus.

20. Verfahren nach einem der Ansprüche 16 bis 19, dadurch gekennzeichnet, daß die Linie der eukaryotischen Zellen selbst eine bestimmte Sequenz von Nukleotiden enthält, die fähig sind, den Teil des Genoms des Adenovirus, von dem der genannte Vektor entnommen ist, zu komplementieren, wobei die genannte Sequenz bevorzugt ins Genom der Zellen der genannten Linie von Zellen inkorporiert ist.

21. Verfahren nach einem der Ansprüche 16 bis 20, dadurch gekennzeichnet, daß die rekombinante DNA die Nukleotidsequenz umfaßt, die dem Gen S des Genoms vom Virus der Hepatitis B entspricht.

22. Verfahren nach einem der Ansprüche 16 bis 20, dadurch gekennzeichnet, daß die rekombinante DNA die Sequenz umfaßt, die dem Gen S und der Region pre-S2 des Genoms vom Virus der Hepatitis B entspricht.

23. Impfstoff dessen aktiver Wirkstoff in einem infektiösen und replizierbaren Adenovirus besteht, umfassend, in eine seiner nicht essentiellen Regionen inkorporiert, insbesondere den nicht essentiellen Teil seiner Region E3, eine rekombinante DNA gebildet zwischen dem frühen Promotor der Region E1A und einer Nuklein-Sequenz der Insertion, die für ein Schutzantigen gegen den gewählten pathogenen Stoff kodiert und insbesondere gegen den Virus der Hepatitis B, wobei diese Nuklein-Sequenz der Insertion die E1A-Gene substituiert, die normalerweise unter der Kontrolle dieses Promotors sind.

FIG.1.

FIG.2.

FIG.3.

FIG.4.

FIG.5.

FIG.6.

EP 0 185 573 B1

pK4(S)

PST I    CLA I

S

+

CLA I                                        Ad 5

2,6                                                                          100

↓

CLA I                              Ad 5(S)

FIG. 7.